# EUROPÄISCHE PATENTSCHRIFT

(11) **EP 1 649 892 B1**
(45) Veröffentlichungstag und Bekanntmachung des Hinweises auf die Patenterteilung: **03.01.2007**
(21) Anmeldenummer: 04025164.7
(22) Anmeldetag: 22.10.2004
(51) Int. Cl.: A61N 2/06

(54) **Stimulationsgerät**
Stimulation device
Appareil de stimulation

(43) Veröffentlichungstag der Anmeldung: 26.04.2006
(73) Patentinhaber: Hahne, Hans Eberhard, 31319 Sehnde (DE)
(72) Erfinder: Hahne, Hans Eberhard, 31319 Sehnde (DE)
(74) Vertreter: Jostarndt, Hans-Dieter

(56) Entgegenhaltungen:
- DE-A1- 3 324 119
- FR-A- 2 603 489
- FR-A- 2 847 803
- US-A1- 2003 092 960
- US-B1- 6 656 108

## Beschreibung

Die vorliegende Erfindung betrifft ein Stimulationsgerät zur Stimulation des menschlichen Körpers mittels eines Magnetfeldes. Insbesondere betrifft die Erfindung ein Stimulationsgerät mit einen in einem Gehäuse angeordneten Permanentmagneten, der in dem Außenraum des Gehäuses ein Magnetfeld erzeugt.

Die Lebensweise von vielen Menschen in der heutigen Zeit ist sehr häufig durch Reizüberflutung und Bewegungsmangel geprägt. Hierzu treten vielfach körperliche Fehlhaltungen oder einseitige körperliche Belastungen am Arbeitsplatz. Diese Faktoren können zu Beeinträchtigungen des Wohlbefindens bei Menschen führen, insbesondere dann, wenn psychische Anspannungen hinzukommen, die beispielsweise durch hohe Erwartungen oder Anforderungen gefördert werden und die genannten ungünstigen Einflussfaktoren zusätzlich verstärken. Die Beeinträchtigungen des Wohlbefindens äußern sich individuell verschieden. Vielfach beobachtet werden in diesem Zusammenhang Kopfschmerzen, Gliederschmerzen, Migräne sowie Rückenschmerzen. Von diesen Symptomen betroffene Menschen suchen naturgemäß nach einer Abhilfe für ihre Leiden. Naheliegend erscheint in diesem Fall zunächst eine medikamentöse Behandlung der Symptome wie zum Beispiel Kopfschmerzen. Allerdings sind dieser Behandlungsmethode wegen der in aller Regel unvermeidlichen Nebenwirkungen von den eingesetzten Medikamenten normalerweise relativ enge zeitliche Grenzen gesetzt. Wegen des ganz erheblichen Nachteils der Nebenwirkungen greifen Betroffene auch zu ganz anderen Methoden wie zum Beispiel autogenem Training, sportlicher Betätigung sowie Akupressurtechniken. Diese letztgenannten Methoden haben den Vorteil, dass sie in aller Regel frei von schädlichen Nebenwirkungen sind. Voraussetzung für den Erfolg dieser letztgenannten Methoden ist allerdings eine entsprechende innere Bereitschaft zum Praktizieren dieser Methoden beziehungsweise eine entsprechende körperliche Konstitution, um beispielsweise ein Schwimmtraining absolvieren zu können. Diese Voraussetzungen sind nicht bei allen Menschen erfüllt.

Hiervon ausgehend ist es eine Aufgabe der vorliegenden Erfindung, ein einfach anwendbares Stimulationsgerät zu schaffen, das dazu geeignet ist, Beeinträchtigungen des Wohlbefindens zu lindern.

Diese Aufgabe wird durch ein Stimulationsgerät nach Anspruch 1 gelöst.

Das erfindungsgemäße Stimulationsgerät weist einen in einem Gehäuse angeordneten Permanentmagnet auf, der in dem Außenraum des Gehäuses ein Magnetfeld erzeugt.

Bei der Erfindung ist das Gehäuse aus Edelmetall hergestellt. Das Edelmetall kann beispielsweise Gold oder Silber sein.

Das Gehäuse ist im Wesentlichen kugelförmig ausgebildet, um eine besonders angenehme Handhabung des Stimulationsgerätes zu erzielen.

Bei einer vorteilhaften Ausführungsform des erfindungsgemäßen Stimulationsgerätes ist das Innere des Gehäuses evakuiert und im Inneren des Gehäuses herrscht ein im Vergleich zum Umgebungsdruck niedrigerer Druck. In Versuchsreihen hat es sich gezeigt, dass zahlreiche Probanten auf diese Ausführungsform besonders günstig reagieren.

In diesem Fall kann es zweckmäßig sein, das Gehäuse mit einer inneren und einer äußeren Schale auszubilden, wodurch es ermöglicht ist, die äußere Schale frei zu gestalten, während die innere Schale für einen gasdichten Abschluss des Innenraumes sorgt.

Bei einer vorteilhaften Ausführungsform des Stimulationsgerätes ist die äußere Schale des Gehäuses aus miteinander verflochtenen Metallbändern hergestellt.

Es hat sich als nützlich erwiesen, in dem Gehäuse mechanische Haltemittel zur Halterung des Permanentmagneten anzuordnen.

Vorteilhafterweise kann der Permanentmagnet zylinderförmig ausgebildet sein, um eine mechanisch einfache und robuste Gestaltung des Stimulationsgerätes zu erreichen.

Zweckmäßigerweise kann der Permanentmagnet als Seltenerdmagnet ausgebildet sein, um ein besonders großes Magnetfeld im Außenraum des Gehäuses zu erzeugen. Beispielsweise kann die Remanenz des Permanentmagneten 1000 bis 1400 mT betragen.

In der Zeichnung ist ein Ausführungsbeispiel der Erfindung dargestellt. Es zeigen:
- Fig. 1:: Das Stimulationsgerät in einer Ansicht von außen,
- Fig. 2a:: das Stimulationsgerät aus Figur 1 in einer aufgeschnittenen Darstellung ohne Permanentmagnet,
- Fig. 2b:: das Stimulationsgerät aus Figur 1 in einer aufgeschnittenen Darstellung mit Permanentmagnet, und
- Fig. 2c:: den Permanentmagnet außerhalb des Stimulationsgerätes.

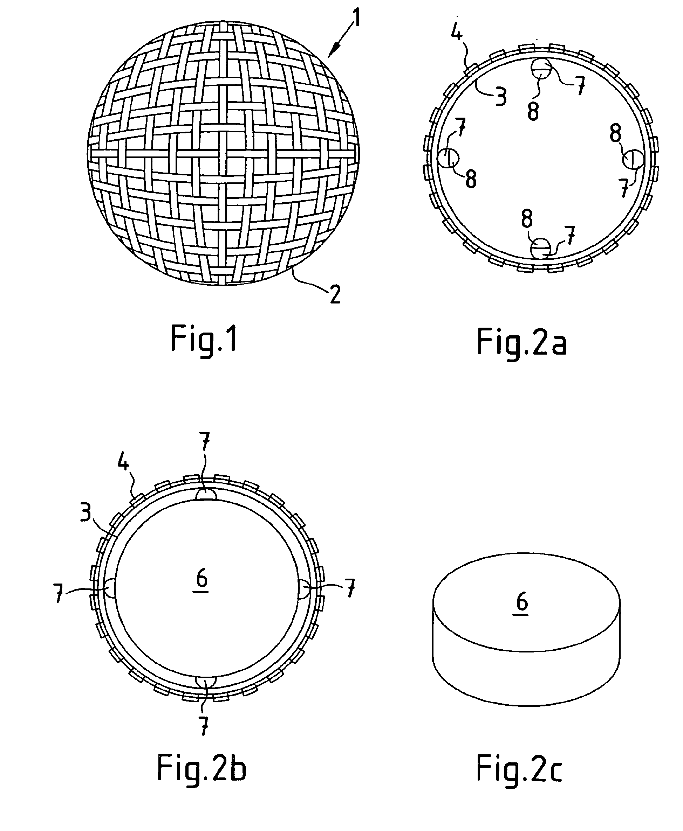

Figur 1 zeigt das als Ganzes mit den Bezugszeichen 1 bezeichnete Stimulationsgerät. Das Stimulationsgerät 1 weist ein Gehäuse 2 auf, welches aus einer inneren und einer äußeren Schale 3, 4 (Figur 2a) aufgebaut ist. Das Gehäuse 2 weist eine strukturierte Oberfläche auf, deren Entstehung weiter unten noch beschrieben werden wird. Das Gehäuse 2 ist aus Edelmetall hergestellt, insbesondere aus Gold. Bei anderen Ausführungsbeispielen kann das Gehäuse 2 auch aus Silber hergestellt sein. Es ist auch möglich, das Gehäuse aus Legierungen aus Gold und Silber zu fertigen. Weiterhin ist es möglich, die innere Schale 2 aus einem anderen Metall beziehungsweise einer anderen Legierung herzustellen wie die äußere Schale. Insbesondere ist es möglich, die äußere Schale außen zu vergolden, während sie unter der Außenoberfläche aus Silber oder aus einer Gold/Silberlegierung hergestellt ist. Darüber hinaus ist es auch möglich, andere Metalle in die Legierung beizumischen um insbesondere eine geforderte mechanische Stabilität des Gehäuses zu erzielen.

In den Figuren 2a und 2b ist das Stimulationsgerät 1 aus Figur 1 in einer aufgeschnittenen Darstellung gezeigt. In Fig. 2a sind im Inneren der inneren Schale 3 vier z-förmige Haltebügel 7 zu erkennen, deren Endlappen 8 als auf Auflage für einen Permanentmagneten 6 (Fig. 2b) dienen. In Fig. 2b ist der zylinderförmige Permanentmagnet 6 zu erkennen, der in dem Gehäuse 2 gehaltert ist. In der in Fig. 2b gezeigten Hälfte des Gehäuses sind ebenso vier z-förmige Haltebügel angeordnet, die jedoch von dem Permanentmagneten 6 in dieser Darstellung fast vollständig verdeckt werden. Der Durchmesser des zylinderförmigen Permanentmagneten 6 und dessen Höhe sowie der Innendurchmesser der inneren Schale 3 sind so aufeinander abgestimmt, dass die kreisförmigen Umfangslinien des Permanentmagneten 6 die Innenoberfläche der inneren Schale 3 fast berühren. In Fig. 2c ist der Permanentmagnet 6 zur besseren Veranschaulichung außerhalb des Gehäuses 2 dargestellt.

Bei dem Permanentmagneten 6 handelt es sich um einen Seltenerdmagneten NdFeB, der eine Remanenz von 1200 mT +/- 100 mT aufweist. Ein solcher Magnet ist kommerziell unter der Bezeichnung Nd35 erhältlich. Die Koerzitivfeldstärke des Permanentmagneten liegt im Bereich von 795 bis 875 kA/m. Das Energieprodukt beträgt 260 bis 285 kJ/m³.

Die Erfindung ist jedoch nicht auf diese Magnetart beschränkt. Bei anderen Ausführungsbeispielen ist es auch möglich, einen anderen Magneten zu verwenden, der ähnliche physikalische Eigenschaften aufweist.

Das insoweit beschriebene Simulationsgerät wird folgendermaßen hergestellt:

Zur Herstellung der äußeren Schale 4 werden zunächst vorbereitete schmale Metallbänder miteinander verflochten, zum Beispiel gemäß einer einfachen sogenannten Leinwandbindung. Das so erzeugte Metallgewebe wird in eine halbkugelförmige vertiefte Form gelegt und mit einem zur Vertiefung passenden Stempel zu einer Halbschale geformt. Danach werden die Ränder beschnitten, damit die Halbschale möglichst genau die Gestalt einer Halbkugel annimmt. Auf diese Weise werden zwei äußere Halbschalen 4 gefertigt.

Hiernach werden aus Blech zwei innere Halbschalen 3 in entsprechender Weise mittels einer kleineren Form gefertigt, wobei der Außendurchmesser der inneren Halbschalen 3 dem Innendurchmesser der äußeren Halbschalen 4 entspricht. Danach werden die inneren und äußeren Halbschalen 3, 4 paarweise ineinander gesetzt und im Randbereich miteinander verbunden. Anschließend werden in die inneren Halbschalen 3 die Haltebügel 7 eingelötet. Jetzt wird in eine der beiden Gehäusehälften 2 der Permanentmagnet eingelegt und dann die andere Gehäusehälfte 2 darauf aufgesetzt. Schließlich werden die beiden Gehäusehälften in einer evakuierten Vorrichtung gasdicht miteinander verschweißt, so dass im Inneren des Gehäuses 2 ein Unterdruck herrscht, der davon abhängt, wie stark die Vorrichtung vor dem Zusammenfügen der Halbkugeln evakuiert wurde.

Die Wirkungen des Stimulationsgerätes lassen sich auf Grundlage der physikalischen und chemischen Eigenschaften des Stimulationsgerätes beschreiben. Um das Stimulationsgerät 1 herum entsteht ein starkes magnetisches Feld, welches magnetische Stoffe und elektrische Ströme in bekannter Weise beeinflusst. Zu den magnetischen Stoffen, die in Zusammenhang mit der Erfindung von großem Interesse sind, gehört in Gefäßen fließendes Blut. Darüber hinaus werden gemäß physikalischer Gesetzmäßigkeiten elektrische Nervenströme und elektrisch geladene Ionen im Körperinneren von dem Stimulationsgerät beeinflusst. Welche physiologischen Wirkungen hiervon hervorgerufen werden, ist im Einzelnen nicht geklärt. Zur Wirkung von Magnetfeldern gibt es allerdings klinische Untersuchungen, die belegen, dass Knochen-, Sehnen- oder Bänderverletzungen unter dem Einfluss von Magnetfeldern schneller heilen als bei herkömmlichen Behandlungsmethoden. Weiterhin werden Einflüsse auf die Zellernährung, die Zellregeneration, die Durchblutung von Blutgefäßen und anderes mehr berichtet.

Neben diesen physikalischen Wirkungen treten bei Probanten auch biochemischen Wirkungen auf, die vermutlich durch die Aufnahme von Goldatomen über die menschliche Haut hervorgerufen werden. Im Bereich der Homöopathie gibt es umfangreiche Literatur über die Wirkungen von Gold. Aufzeichnungen über die medizinische Wirkung von Gold gehen bis auf Paracelsus und Hildegard von Bingen zurück. Beispielsweise ist es bekannt, dass unter die Haut implantierte Goldplättchen eine erhebliche Linderung der Beschwerden bei Arthritis und Arthrose herbeiführen. In diesem Zusammenhang kommt der stark strukturierten Oberfläche der äußeren Schale eine besondere Bedeutung zu, denn hierdurch wird bereichsweise ein besonders intensiver Kontakt zwischen dem Stimulationsgerät und den Berührungszonen auf der Haut geschaffen. Darüber hinaus führt die Oberflächenstruktur zu einer örtlichen Variation der magnetischen Permeabilität, woraus örtliche Veränderungen des Magnetfeldes entstehen.

Ebenso wie Gold werden auch Silber in der Homöopathie medizinische Wirkungen zugeschrieben. Die Wirkmechanismen mit Bezug auf das Stimulationsgerät sind bei Silber dieselben wie bei Gold, d.h. der intensive Hautkontakt spielt die entscheidende Rolle.

In der praktischen Anwendung wird das Stimulationsgerät beispielsweise auf bekannte Akupressurpunkte aufgelegt. Dort kann es mit der Hand hin- und hergerollt werden, was durch die kugelförmige Gestalt des Stimulationsgerätes erleichtert wird. Hierbei ist zu beachten, dass sich durch diese Bewegung das im Körper des Benutzers wirkende Magnetfeld in Richtung und Stärke ändert, weil der Permanentmagnet fest in der Kugel gehaltert ist und ein statisches Magnetfeld im Außenraum des Gehäuses 2 erzeugt. Eine rollende Bewegung des Stimulationsgerätes führt folglich zu der genannten Änderung der Richtung und Stärke des Magnetfeldes an einem bestimmten Punkt des behandelten Körpers. In Versuchsreihen hat es sich gezeigt, dass es zahlreiche Körperstellen gibt, unter anderem die Handflächen, wo die Anwendung des Stimulationsgerätes zu einer deutlichen Verbesserung des Wohlbefindens führt. Probanten berichten zum Beispiel von einer raschen Befreiung von Kopfschmerzen, Gliederschmerzen und sogar Menstruationsbeschwerden.

Die Gesamtheit der vorliegenden Beobachtungen deutet darauf hin, dass das erfindungsgemäße Stimulationsgerät in der Lage ist, zu einer erheblichen Steigerung des Wohlbefindens bei Menschen zu führen.

## Patentansprüche

1. Stimulationsgerät mit einem in einem kugelförmigen Gehäuse (2) angeordneten Permanentmagnet (6), der in dem Außenraum des Gehäuses (2) ein Magnetfeld erzeugt, **dadurch gekennzeichnet, dass** das Gehäuse (2) aus Edelmetall hergestellt ist.

2. Stimulationsgerät nach Anspruch 1, **dadurch gekennzeichnet, dass** das Edelmetall Gold oder Silber umfasst.

3. Stimulationsgerät nach Anspruch 1 oder 2, **dadurch gekennzeichnet, dass** das Innere des Gehäuses (2) evakuiert ist und im Inneren des Gehäuses ein im Vergleich zum Umgebungsdruck niedrigerer Druck herrscht.

4. Stimulationsgerät nach einem der vorstehenden Ansprüche, **dadurch gekennzeichnet, dass** das Gehäuse (2) eine innere und eine äußere Schale (3, 4) umfasst.

5. Stimulationsgerät nach Anspruch 4, **dadurch gekennzeichnet, dass** die innere Schale (3) des Gehäuses (2) gasdicht ausgebildet ist.

6. Stimulationsgerät nach Anspruch 4, **dadurch gekennzeichnet, dass** die äußere Schale (4) des Gehäuses (2) aus miteinander verflochtenen Metallbändern hergestellt ist.

7. Stimulationsgerät nach einem oder mehreren der vorstehenden Ansprüche, **dadurch gekennzeichnet, dass** in dem Gehäuse (2) mechanische Haltemittel (7, 8) zur Halterung des Permanentmagneten (6) angeordnet sind.

8. Stimulationsgerät nach Anspruch 1, **dadurch gekennzeichnet, dass** der Permanentmagnet (6) zylinderförmig ausgebildet ist.

9. Stimulationsgerät nach Anspruch 1, **dadurch gekennzeichnet, dass** der Permanentmagnet (6) als Seltenerdmagnet ausgebildet ist.

10. Stimulationsgerät nach Anspruch 11, **dadurch gekennzeichnet, dass** die Remanenz des Permanentmagneten (6) 1000 bis 1400 mT beträgt.

## Claims

1. A stimulation device with a permanent magnet (6) that is arranged in a spherical housing (2) and that generates a magnetic field in the outer chamber of the housing (2), **characterized in that** the housing (2) is made of a precious metal.

2. The stimulation device according to Claim 1, **characterized in that** the precious metal is gold or silver.

3. The stimulation device according to Claim 1 or 2, **characterized in that** the inside of the housing (2) is evacuated and, inside the housing, a pressure prevails that is lower than the ambient pressure.

4. The stimulation device according to any of the preceding claims, **characterized in that** the housing (2) comprises an inner and an outer shell (3, 4).

5. The stimulation device according to Claim 4, **characterized in that** the inner shell (3) of the housing (2) is configured so as to be gas-tight.

6. The stimulation device according to Claim 4, **characterized in that** the outer shell (4) of the housing (2) is made of interwoven metal strips.

7. The stimulation device according to one or more of the preceding claims, **characterized in that** mechanical holding means (7, 8) for holding the permanent magnet (6) are arranged in the housing (2).

8. The stimulation device according to Claim 1, **characterized in that** the permanent magnet (6) is configured so as to be cylindrical.

9. The stimulation device according to Claim 1, **characterized in that** the permanent magnet (6) is configured as a rare-earth magnet.

10. The stimulation device according to Claim 11 *[sic],* **characterized in that** the remanent magnetization of the permanent magnet (6) is 1000 to 1400 mT.

## Revendications

1. Appareil de stimulation avec un aimant permanent (6) disposé dans un boîtier sphérique (2) qui génère dans la chambre externe du boîtier (2) un champ magnétique **caractérisé en ce que** le boîtier (2) se compose de métal précieux.

2. Appareil de stimulation selon la revendication 1, **caractérisé en ce que** le métal précieux est de l'or ou de l'argent.

3. Appareil de stimulation selon la revendication 1 ou 2, **caractérisé en ce que** l'intérieur du boîtier (2) est mis sous vide et une pression plus faible que la pression environnante règne à l'intérieur du boîtier.

4. Appareil de stimulation selon l'une des revendications précédentes, **caractérisé en ce que** le boîtier (2) comprend une coque interne et externe (3, 4).

5. Appareil de stimulation selon la revendication 4, **caractérisé en ce que** la coque interne (3) du boîtier (2) est réalisée étanche au gaz.

6. Appareil de stimulation selon la revendication 4, **caractérisé en ce que** la coque externe (4) du boîtier (2) est fabriquée à partir de bandes de métal tressées entre elles.

7. Appareil de stimulation selon l'une des revendications précédentes, **caractérisé en ce que** des moyens de retenue (7, 8) mécaniques sont disposés dans le boîtier (2) pour le maintien de l'aimant permanent (6).

8. Appareil de stimulation selon la revendication 1, **caractérisé en ce que** l'aimant permanent (6) est de forme cylindrique.

9. Appareil de stimulation selon la revendication 1, **caractérisé en ce que** l'aimant permanent est réalisé comme un aimant en terre rare.

10. Appareil de stimulation selon la revendication 1, **caractérisé en ce que** la rémanence de l'aimant permanent (6) est comprise entre 1000 et 1400 mT.
